# EUROPEAN PATENT APPLICATION

(11) **EP 3 973 844 A1**
(43) Date of publication of application: **30.03.2022**
(21) Application number: 21197013.2
(22) Date of filing: 16.09.2021
(51) Int. Cl.: A61B 1/018, A61B 1/00, A61B 17/00

(54) **TREATMENT TOOL DEVICE FOR ENDOSCOPE AND ENDOSCOPE SYSTEM**

(30) Priority: 23.09.2020 JP 2020158526
(71) Applicant: FUJIFILM Corporation, Tokyo 106-8620 (JP)
(72) Inventor: DEJIMA, Takumi, Kanagawa, 258-8538 (JP)
(74) Representative: Klunker IP Patentanwälte PartG mbB

(57) **Abstract**

Provided are a treatment tool device for an endoscope and an endoscope system capable of increasing the number of the types of treatment tool operations that can be performed by a doctor or an assistant, to perform the treatment tool operations without the need for cooperation, and capable of reducing the number of people who perform the treatment.

A treatment tool device for an endoscope includes a treatment tool main body and an oversheath. The treatment tool main body includes a flexible sheath, an operation wire, a snare wire, and a hard part. The operation wire is inserted through the flexible sheath. The hard part is provided at a base end portion of the flexible sheath. The oversheath is inserted into a forceps channel in a state where the flexible sheath and the hard part are inserted into the oversheath.

## Description

### BACKGROUND OF THE INVENTION

### 1. Field of the Invention

The present invention relates to a treatment tool device for an endoscope and an endoscope system that are used by being inserted through a forceps channel of an endoscope.

### 2. Description of the Related Art

In the medical field, an insertion part of an endoscope is inserted into the body of a subject to perform not only observation of an inside of the body but also various treatments on an observation site. Specifically, various treatment tools, such as forceps and an incision tool, are inserted through a forceps channel in the insertion part from a forceps port provided in an operation part of the endoscope, and are led out from a forceps outlet that is open at a distal end of the insertion part, whereby various treatments, such as resection and collection of the observation site, are performed.

JP4663345B discloses a treatment tool for an endoscope comprising a flexible sheath, a treatment tool main body, and an attachment part for attaching the flexible sheath to an operation part of the endoscope. The flexible sheath is inserted into a body cavity through a forceps channel of the endoscope. The treatment tool main body is formed of a flexible operation wire, a treatment part provided at a distal end of the operation wire, and a hard pipe connected to a base end of the operation wire. The operation wire and the pipe are supported by the flexible sheath so as to advance and retreat. The treatment part is a snare wire that contracts in a case of being housed inside the flexible sheath and expands in a case of protruding from a distal end of the flexible sheath. Thus, an operation of expanding the treatment part by causing the treatment part to protrude from the distal end of the flexible sheath inserted into the body cavity can be performed near the operation part of the endoscope.

### SUMMARY OF THE INVENTION

In the medical field, further preventive measures against infectious diseases are desired. Therefore, it is required to reduce the risk of infection by performing various treatments using an endoscope and a treatment tool with as few people as possible. In addition, due to problems such as a shortage of medical personnel and an increase in burden of medical expenses, it is desired to perform a treatment with a small number of people. In a case where there is an assistant who operates the treatment tool in addition to a doctor who operates the endoscope, there is a problem that the treatment cannot be performed smoothly unless the cooperation between the doctor and the assistant is good.

However, in the treatment tool for an endoscope disclosed in JP4663345B, in a case where the doctor operates the endoscope, an operation of the treatment tool for an endoscope that can be performed by the doctor is only an operation of advancing and retreating the treatment tool to and from the forceps channel. That is, operations other than the operation of advancing and retreating the treatment tool to and from the forceps channel, for example, an operation of expanding or contracting the treatment part or an operation of rotating the treatment tool around a central axis with respect to the forceps channel, require assistance from the assistant, and the doctor needs cooperation of the operations, such as issuing instructions to the assistant. Moreover, the number of people who perform a treatment cannot be reduced.

An object of the present invention is to provide a treatment tool device for an endoscope and an endoscope system capable of increasing the number of the types of treatment tool operations that can be performed by either a doctor who operates an endoscope or an assistant, to perform the treatment tool operations without the need for cooperation, and capable of reducing the number of people who perform the treatment in a case where the doctor is a main operator.

A treatment tool device for an endoscope according to an aspect of the present invention comprises a treatment tool main body and an oversheath. The treatment tool main body comprises a flexible sheath having a tubular shape and inserted into a forceps channel of an endoscope, an operation wire inserted through the flexible sheath, a hard part provided at a base end portion of the flexible sheath and harder than the flexible sheath, and an operation part continuously provided at the hard part. The oversheath is inserted into a forceps channel in a state where the flexible sheath and the hard part are inserted into the oversheath.

It is preferable that the oversheath has a fitting part that is fitted to the hard part, and that the treatment tool main body is slidable and rotatable with respect to the oversheath within a range in which the hard part and the fitting part are fitted to each other.

It is preferable that a length of the oversheath is shorter than a length of the flexible sheath. It is preferable that the oversheath is provided with a seal member filling a gap between the oversheath and the treatment tool main body at a base end portion. It is preferable that the seal member is an annular elastic member housed inside the oversheath.

It is preferable that in a case where a distal end of the operation part is made to abut on a base end of the oversheath, a length of the flexible sheath protruding from a distal end of the oversheath is 40 cm or more and 270 cm or less.

It is preferable that the oversheath is provided with an index indicating an insertion length of the oversheath inserted into the forceps channel. It is preferable that a mark is used as the index indicating the insertion length, and that a dimension from a distal end of the oversheath to the mark is 4 cm. Alternatively, it is preferable that gradations indicating a dimension from a distal end of the oversheath are used as the index indicating the insertion length.

It is preferable that the oversheath has an oversheath main body that is inserted into the forceps channel and a stopper provided on the oversheath main body to regulate insertion of the oversheath, and it is more preferable that a dimension from a distal end of the oversheath main body to the stopper is 4 cm.

An endoscope system according to an aspect of the present invention comprises an endoscope provided with a forceps channel and a forceps valve, and the treatment tool device for an endoscope.

An endoscope system according to an aspect of the present invention comprises an endoscope provided with a forceps channel, a forceps valve, and a suction channel connected to the forceps channel, and the treatment tool device for an endoscope, in which the treatment tool device for an endoscope is inserted into the forceps channel in a state where the oversheath does not reach a branch part between the forceps channel and the suction channel.

It is preferable that the endoscope is provided with an attaching/detaching member that makes the oversheath attachable and detachable. It is preferable that a first resistance force in a case where the oversheath is advanced and retreated to and from the forceps valve is greater than a second resistance force in a case where the treatment tool main body is advanced and retreated to and from the oversheath.

According to the aspects of the present invention, it is possible to increase the number of the types of treatment tool operations that can be performed by either a doctor who operates an endoscope or an assistant, to perform the treatment tool operations without the need for cooperation, and is possible to reduce the number of people who perform the treatment in a case where the doctor is a main operator.

### BRIEF DESCRIPTION OF THE DRAWINGS

Fig. 1 is a schematic view showing an endoscopic examination using an endoscope and a treatment tool device for an endoscope.
Fig. 2 is a front view of the endoscope and the treatment tool device for an endoscope.
Fig. 3 is a front view of the treatment tool device for an endoscope.
Figs. 4A and 4B are explanatory views of a state in which a snare wire is expanded and a state in which the snare wire is contracted.
Fig. 5 is a cross-sectional view of a main part of an oversheath.
Fig. 6 is an exploded perspective view showing a configuration of the oversheath.
Fig. 7 is a perspective view of the treatment tool device for an endoscope.
Fig. 8 is a perspective view showing a state before the treatment tool device for an endoscope is attached to the endoscope.
Fig. 9 is an explanatory view showing an operation of performing a treatment using the treatment tool device for an endoscope.
Figs. 10A and 10B are explanatory views of treating a lesion portion using the treatment tool device for an endoscope, which shows a state of Fig. 10A in which a distal end portion of a flexible sheath is aligned with a position of the lesion portion and a state of Fig. 10B in which a snare wire is made to protrude.
Fig. 11 is an explanatory view Figs. 11A and 11B are explanatory views of treating the lesion portion using the treatment tool device for an endoscope, which shows a state of Fig. 11A in which the lesion portion is surrounded by a snare wire and a state of Fig. 11B in which the lesion portion is resected.
Fig. 12 is a front view of a treatment tool device for an endoscope of a second embodiment.

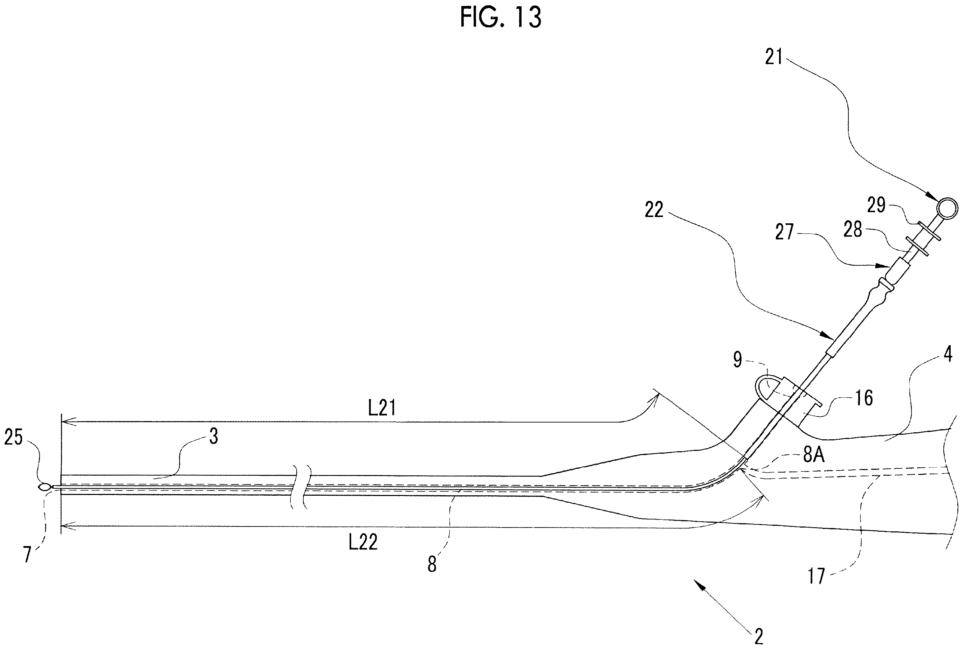

Fig. 13 is an explanatory view showing a dimensional relationship between the treatment tool device for an endoscope of the second embodiment and a forceps channel of the endoscope.
Fig. 14 is a front view of a treatment tool device for an endoscope of a first modification example.
Fig. 15 is an explanatory view showing a dimensional relationship between the treatment tool device for an endoscope of the first modification example and the forceps channel of the endoscope.
Fig. 16 is a front view of a treatment tool device for an endoscope of a second modification example.
Fig. 17 is a front view of a treatment tool device for an endoscope of a third modification example.
Fig. 18 is an explanatory view showing an operation of performing an endoscopic examination and treatment in a fourth modification example.
Fig. 19 is an explanatory view showing an operation of performing an endoscopic examination and treatment in a fifth modification example.

### DESCRIPTION OF THE PREFERRED EMBODIMENTS

### First Embodiment

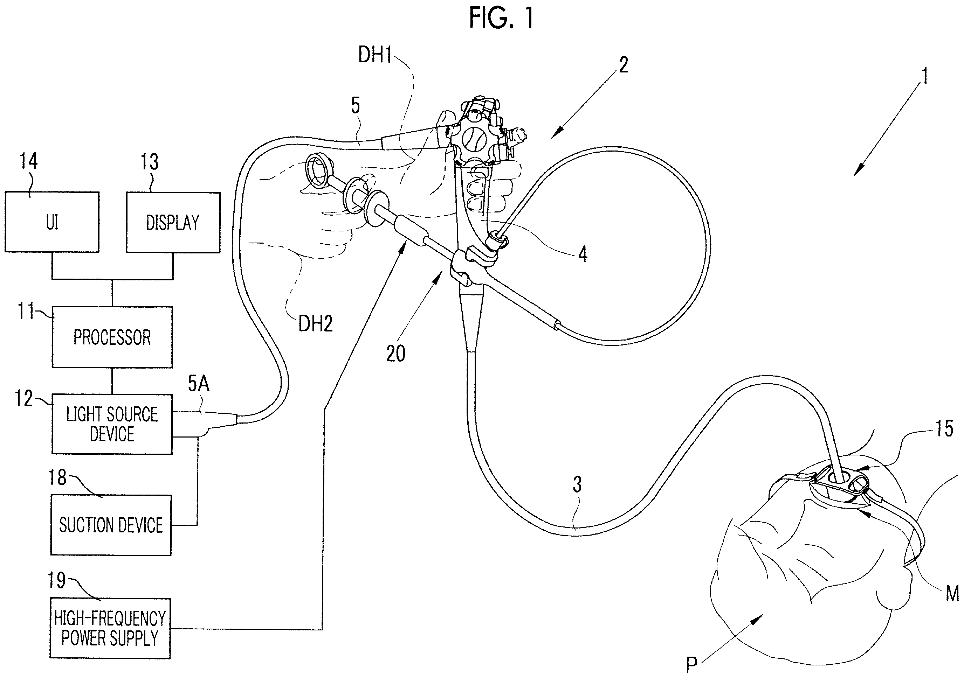

As shown in Fig. 1, an endoscope system 1 according to the embodiment of the present invention comprises an endoscope 2, a processor device 11, a light source device 12, a display 13, a user interface (UI) 14, a suction device 18, a high-frequency power supply 19, and a treatment tool device 20 for an endoscope. The endoscope 2 is, for example, an upper digestive tract endoscope for an esophagus, a stomach, or the like, and comprises an insertion part 3 inserted into an upper digestive tract of a patient P as a subject, an operation part 4 continuously provided at a base end portion of the insertion part 3, a universal cord 5 connected to the operation part 4.

The universal cord 5 is connected to an external device such as the processor device 11 or the light source device 12 via a connector 5A. The processor device 11 is electrically connected to the display 13 and the UI 14. The UI 14 has a keyboard, a mouse, a touch pad, a microphone, and the like, and accepts an input operation of a doctor D who is a user.

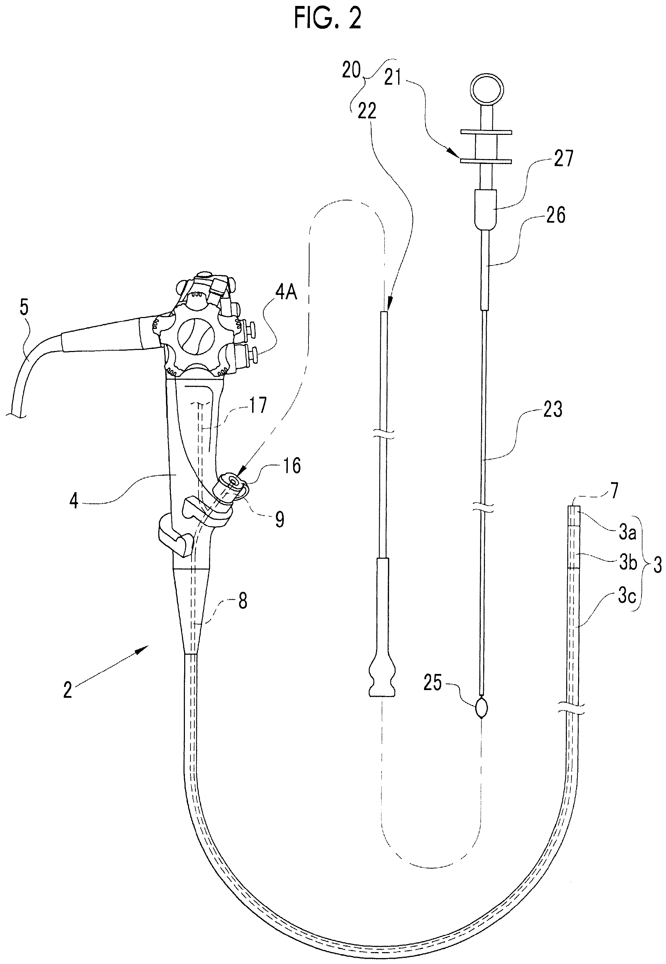

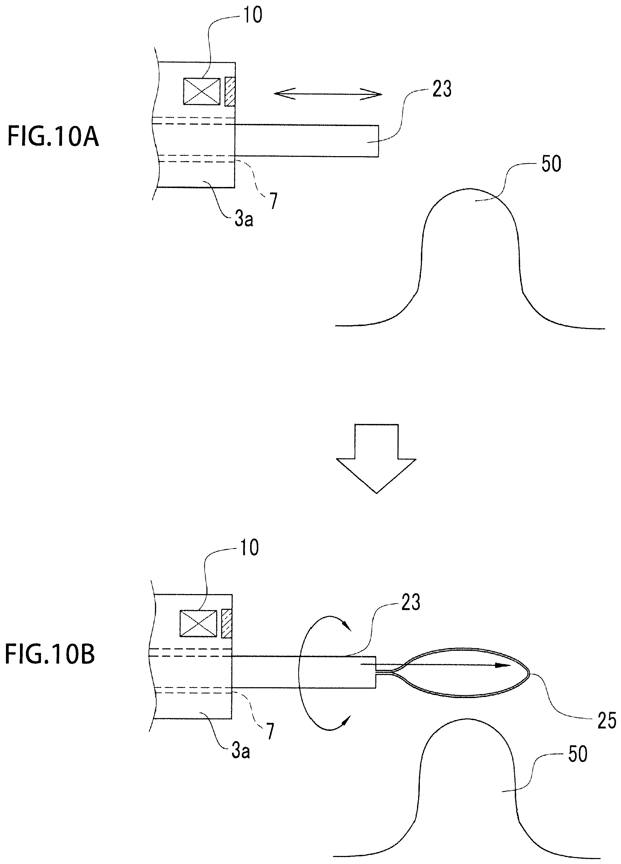

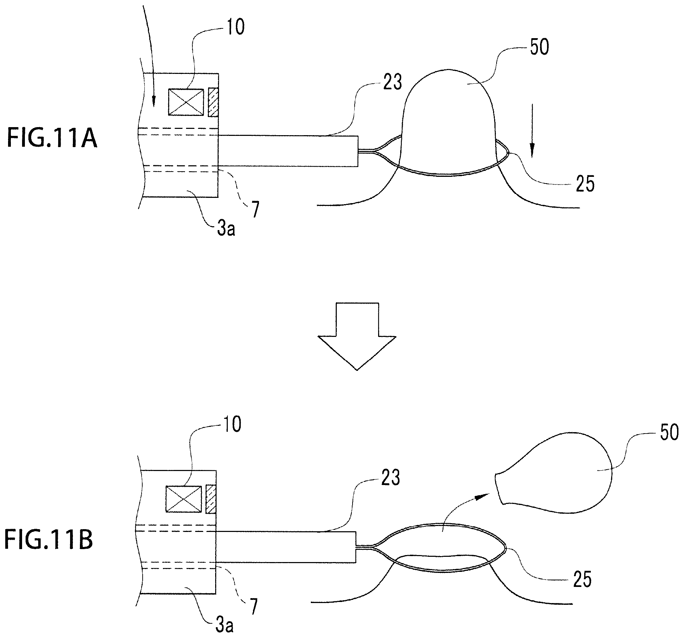

As shown in Fig. 2, the insertion part 3 consists of a distal end rigid portion 3a, a bending portion 3b, and a flexible tube portion 3c having flexibility in this order from a distal end side to a base end side. An observation window and an illumination window are provided on a distal end surface of the distal end rigid portion 3a, although not shown. An image sensor 10 (see Figs. 10 and 11) or the like is disposed on the inside of the observation window, and an optical fiber cable (not shown) is disposed on the inside of the illumination window. A signal line of the image sensor and the optical fiber cable are respectively connected to the processor device 11 and the light source device 12 through the insertion part 3, the operation part 4, the universal cord 5, and the connector 5A. The processor device 11 performs image processing or the like on an endoscope image captured by the image sensor 10 and displays it on the display 13. The bending portion 3b is continuously provided at the distal end rigid portion 3a and is provided so as to be bendable.

In a case where the insertion part 3 of the endoscope 2 is inserted from a mouth M of the patient P, a mouthpiece 15 for an endoscope is mounted on the mouth of the patient P. The mouthpiece 15 for an endoscope has a pipe line (not shown) through which the insertion part 3 is inserted. The mouthpiece 15 for an endoscope is mounted on the mouth M of the patient P by inserting a part of the mouthpiece into the mouth M of the patient P and holding the inserted portion by the patient P. Thus, the insertion part 3 can be introduced into the body through the pipe line.

A forceps channel 8 through which a treatment tool main body 21 and an oversheath 22, which will be described below, are inserted is disposed in the insertion part 3. One end of the forceps channel 8 is connected to a forceps outlet 7, and the other end thereof is connected to a forceps port 9 provided in the operation part 4. The forceps port 9 is provided with a forceps valve 16. The forceps channel 8 is also used as a route for feeding a cleaning liquid such as water from the forceps outlet 7 and a route for sucking a body fluid such as blood and contents such as body waste materials. A suction channel 17 branched from the forceps channel 8 is disposed in the operation part 4, and one end of the suction channel 17 is connected to the forceps channel 8 and the other end thereof is connected to an operation button 4A provided on the operation part 4.

The operation button 4A comprises a suction valve (not shown) provided inside. The suction valve is connected to the suction channel 17 in the operation part 4, and is connected to the external suction device 18 via a pipe line (not shown) disposed in the operation part 4, the universal cord 5, and the connector 5A. The suction device 18 is, for example, a suction pump that generates a negative pressure. By pressing a pressing portion of the operation button 4A, the suction channel 17 communicates with a pipe line of the suction device 18. As a result, a body fluid or the like can be sucked from the forceps outlet 7 of the insertion part 3 inserted into the subject. In addition, by releasing the pressing operation of the pressing portion, the communication between the suction channel 17 and the pipe line of the suction device 18 is interrupted, and the suction from the forceps outlet 7 can be stopped.

The high-frequency power supply 19 causes a high-frequency current to flow through a snare wire 25, which will be described below, of the treatment tool device 20 for an endoscope. As a result, the lesion portion can be resected.

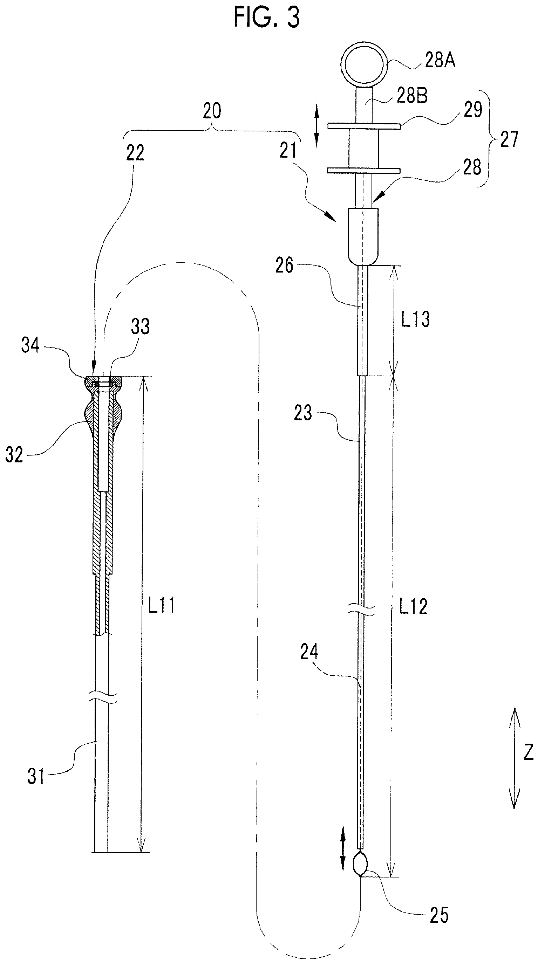

As shown in Fig. 3, the treatment tool device 20 for an endoscope comprises the treatment tool main body 21 and the oversheath 22. The treatment tool main body 21 is, for example, a high-frequency snare for resecting the lesion portion by causing a high-frequency current to flow therethrough. The treatment tool main body 21 comprises a flexible sheath 23, an operation wire 24, the snare wire 25 serving as a treatment part, a hard part 26, and an operation part 27. The flexible sheath 23 is a tubular sheath formed of a flexible material, for example, a soft resin, and is inserted into the forceps channel 8 of the endoscope 2 together with the oversheath 22. The operation wire 24 is provided integrally with the snare wire 25 and is inserted through the flexible sheath 23.

The hard part 26 is provided at a base end portion of the flexible sheath 23 and is formed of a material harder than that of the flexible sheath 23, for example, a hard resin. The hard part 26 is disposed coaxially with the flexible sheath 23, that is, on a central axis parallel to an insertion direction Z into the forceps channel 8. The hard part 26 is formed in a cylindrical shape and is fitted to fitting portions 31B and 32B to be described below.

The operation part 27 comprises an operation part main body 28 and a slider 29 supported by the operation part main body 28 to be slidable. As described above, the hard part 26 is provided at the base end portion of the flexible sheath 23, and the operation part main body 28 is continuously provided at a base end portion of the hard part 26.

The operation part main body 28 is provided with a finger hook portion 28A and a cylindrical portion 28B parallel to the insertion direction Z. The slider 29 is engaged with the cylindrical portion 28B and slides along the cylindrical portion 28B in an axial direction of the flexible sheath 23. In a case where the patient P is treated, a thumb of the user is hooked on the finger hook portion 28A, and an index finger and a middle finger of the same user are hooked on the slider 29. A base end of the operation wire 24 is fixed to the slider 29. Therefore, the operation wire 24 is pushed and pulled in the flexible sheath 23 in the axial direction with the sliding movement of the slider 29.

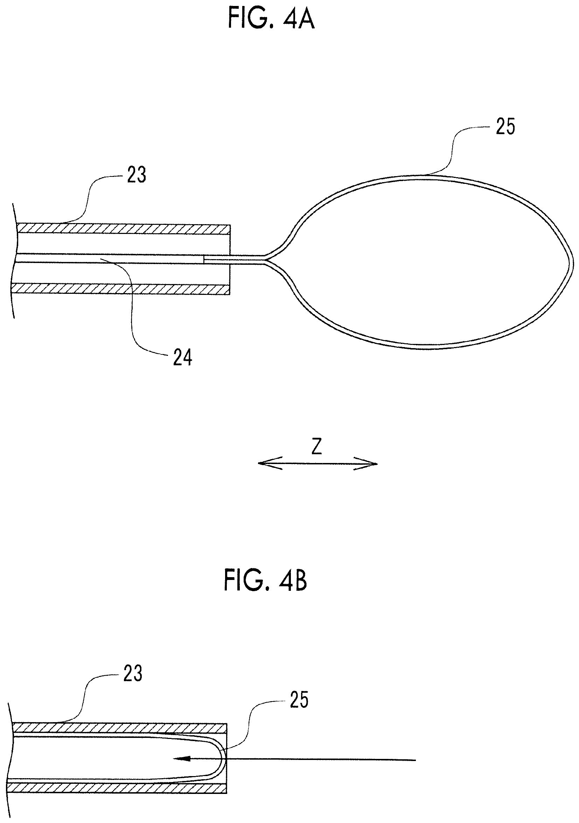

As shown in Fig. 4A, the snare wire 25 expands in a loop shape in a case of protruding from a distal end of the flexible sheath 23 in the insertion direction Z due to the push-pull operation of the operation wire 24. Thus, the snare wire 25 can surround the lesion portion. On the other hand, as shown in Fig. 4B, in a case where the snare wire 25 is housed inside the flexible sheath 23 by the push-pull operation of the operation wire 24 accompanying the sliding movement of the slider 29, the snare wire 25 is in a contracted state.

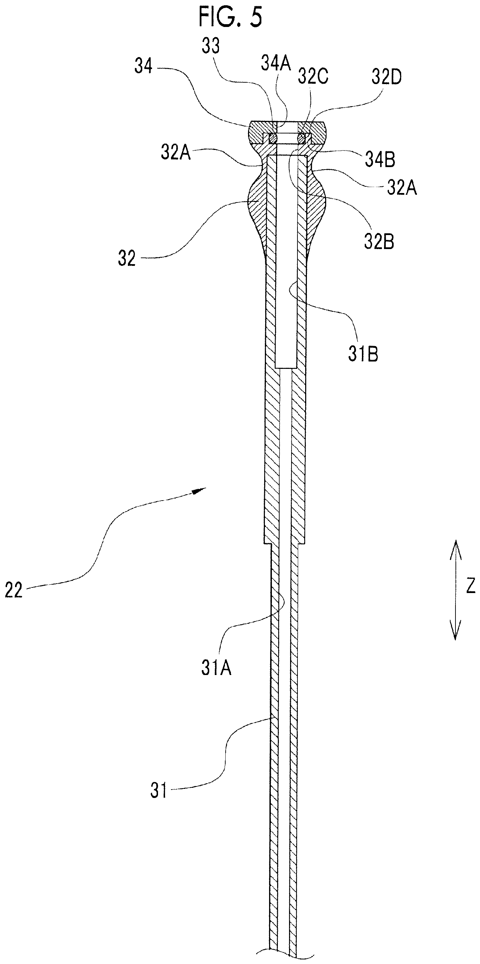

As shown in Fig. 5, in the oversheath 22, an oversheath main body 31, a seal holding member 32, a seal member 33, and a lid member 34 are integrally provided. The oversheath main body 31 is a tubular sheath formed of a flexible material, for example, a soft resin, and is inserted into the forceps channel 8 of the endoscope 2.

The oversheath main body 31 has a pipe line 31A and a fitting portion 31B. The flexible sheath 23 of the treatment tool main body 21 is inserted into the pipe line 31A. The fitting portion 31B communicates with the pipe line 31A and has an inner diameter larger than that of the pipe line 31A. The fitting portion 31B is fitted to the hard part 26 of the treatment tool main body 21 to be slidable and rotatable. In the oversheath 22, except for the seal member 33, the oversheath main body 31, the seal holding member 32, and the lid member 34, are formed of a soft material such as a soft resin.

The seal holding member 32 is fixed to a base end portion of the oversheath main body 31 in the insertion direction Z, and protrudes from an outer peripheral surface of the oversheath main body 31. The seal holding member 32 has a pair of recess portions 32A, the fitting portion 32B, an opening portion 32C, and an engaging projection portion 32D. The pair of recess portions 32A is a concave portion in an outer shape of the seal holding member 32, and is constricted in a direction orthogonal to the insertion direction Z. The recess portion 32A is engaged with an attaching/detaching member 40 to be described below. The fitting portion 32B communicates with the fitting portion 31B of the oversheath main body 31. The fitting portion 32B is fitted to the hard part 26 of the treatment tool main body 21 to be slidable and rotatable in the same manner as the fitting portion 31B.

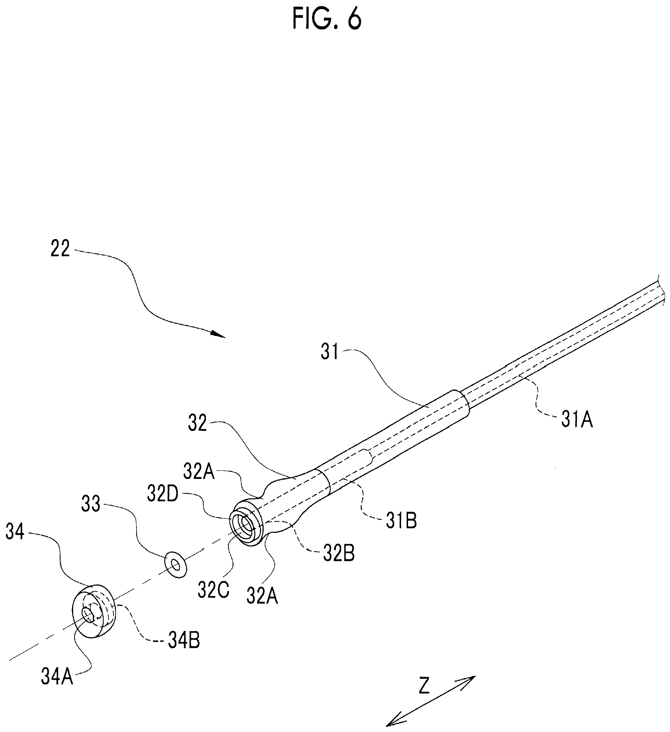

As shown in Fig. 6, the opening portion 32C is formed at a base end of the fitting portion 32B in the insertion direction Z. The opening portion 32C is a circular opening portion formed to match an outer shape of the seal member 33. The seal member 33 is formed of an annular elastic member, for example, a rubber member. The seal member 33 is housed inside the opening portion 32C. That is, the seal member 33 is provided at a base end portion of the oversheath 22 and fills a gap between the oversheath 22 and the treatment tool main body 21. Thus, in various treatments, airtightness between the treatment tool main body 21 and the oversheath 22 is improved, air and carbon dioxide in the stomach are prevented from escaping, and various treatments can be performed in a comfortable space for a doctor or an assistant.

An edge surface shape of a base end portion of the seal holding member 32 in the insertion direction Z is circular or elliptical. The engaging projection portion 32D is a cylindrical projection portion disposed around the opening portion 32C and protruding toward the base end side in the insertion direction Z.

The lid member 34 is formed in a shape that is the same as or one size larger than the base end portion of the seal holding member 32. The lid member 34 has a through-hole 34A and an engaging recess portion 34B. The through-hole 34A is a circular through-hole located at the center of the lid member 34. The through-hole 34A has the same inner diameter as the fitting portions 31B and 32B.

The engaging recess portion 34B is formed on a side facing the seal holding member 32, that is, on an edge surface of the lid member 34 on the distal end side in the insertion direction Z. The engaging recess portion 34B is a circular recess portion that matches an outer shape of the engaging projection portion 32D of the seal holding member 32. The engaging recess portion 34B is engaged with the engaging projection portion 32D, thereby coupling the lid member 34 to the seal holding member 32. In this case, the seal member 33 is interposed between the lid member 34 and the seal holding member 32. Thus, the movement of the seal member 33 in the insertion direction Z is regulated, and the seal member 33 is fixed inside the seal holding member 32.

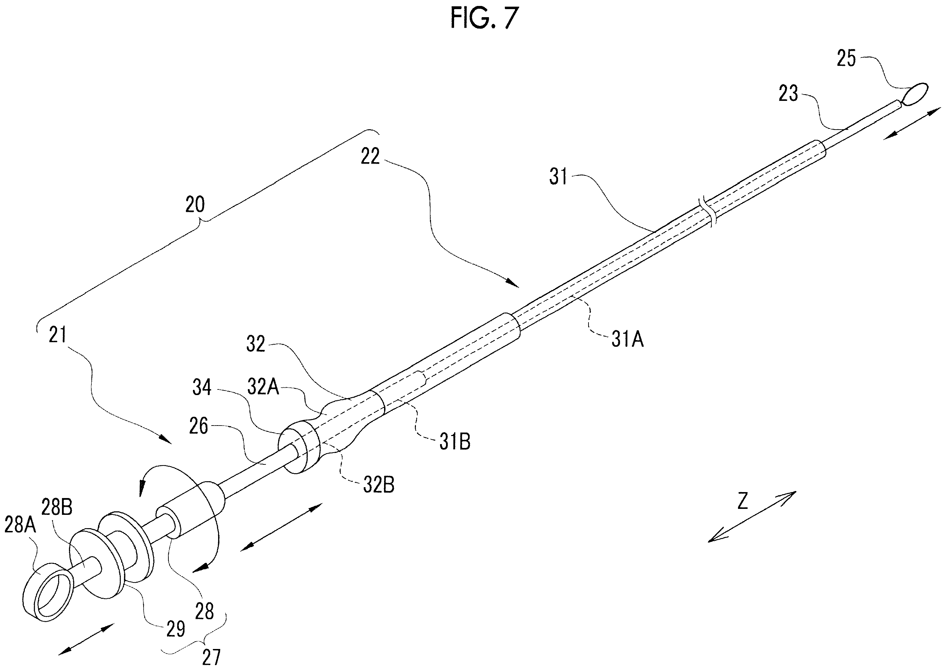

As shown in Fig. 7, in the oversheath 22, the flexible sheath 23 is inserted into the pipe line 31A, and the hard part 26 is inserted into the fitting portions 31B and 32B. The treatment tool main body 21 is slidable and rotatable with respect to the oversheath 22 within a range in which the hard part 26 and the fitting portions 31B and 32B are fitted to each other. Further, in this state, the snare wire 25 can be brought into the expanded state or the contracted state by the push-pull operation of the operation wire 24 accompanying the sliding movement of the slider 29.

An outer shape of the operation part 27 of the treatment tool main body 21 is larger than an outer shape of the hard part 26. Therefore, in a case where the treatment tool main body 21 is slid toward the distal end side in the insertion direction Z with respect to the oversheath 22, the operation part 27 abuts against the base end of the oversheath 22. Thus, the user can recognize that the treatment tool main body 21 cannot be slid further toward the distal end side in the insertion direction Z with respect to the oversheath 22.

A length L11 (see Fig. 3) of the oversheath 22 in the insertion direction Z is shorter than a length L12 (see Fig. 3) of the flexible sheath 23. As a result, in a case where the flexible sheath 23 and the hard part 26 are inserted into the oversheath 22, the distal end of the flexible sheath 23 protrudes from the distal end of the oversheath 22. Therefore, in a case where the operation wire 24 is pushed and pulled, the snare wire 25 does not come into contact with the oversheath 22, and the snare wire 25 can be brought into the expanded state. In addition, a length L13 of the hard part 26 in the insertion direction Z is preferably 70 mm or more and 100 mm or less. Thus, a range in which the hard part 26 and the fitting portions 31B and 32B are fitted to each other can be sufficiently secured, and the treatment tool main body 21 can be easily slid and rotated with respect to the oversheath 22.

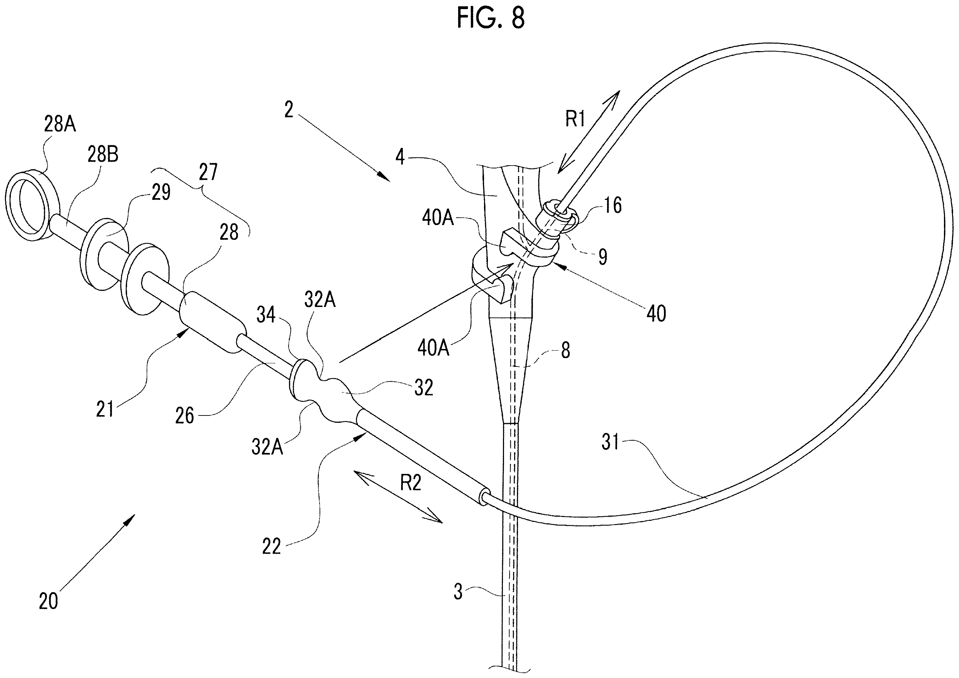

As shown in Fig. 8, the treatment tool device 20 for an endoscope is inserted into the forceps channel 8 of the endoscope 2 in a state where the flexible sheath 23 and the hard part 26 are inserted into the oversheath 22. Specifically, the oversheath 22 is inserted from the forceps port 9 into the forceps channel 8 through a slit valve (not shown) formed inside the forceps valve 16.

The attaching/detaching member 40 is provided on the operation part 4 of the endoscope 2. The attaching/detaching member 40 has a pair of projection portions 40A facing each other. The projection portion 40A is formed so as to match the recess portion 32A of the oversheath. By engaging the projection portion 40A with the recess portion 32A, the attaching/detaching member 40 can attach the oversheath 23 attachably and detachably.

In the endoscope system 1, in a case where a first resistance force in a case where the oversheath 22 is advanced and retreated to and from the forceps valve 16 is defined as R1 and a second resistance force in a case where the treatment tool main body 21 is advanced and retreated to and from the oversheath 22 is defined as R2, the first resistance force R1 is greater than the second resistance force R2. Here, the first resistance force R1 is a frictional force mainly generated between the forceps valve 16 and the oversheath main body 31, and the second resistance force R2 is a frictional force mainly generated between the seal member 33 of the oversheath 22 and the hard part 26 of the treatment tool main body 21. In order to make the first resistance force R1 greater than the second resistance force R2, for example, a material having a smooth surface (small friction) need only be selected as the seal member 33, or a dimensional difference between an outer diameter of the hard part 26 and an inner diameter of the seal member 33 need only be adjusted.

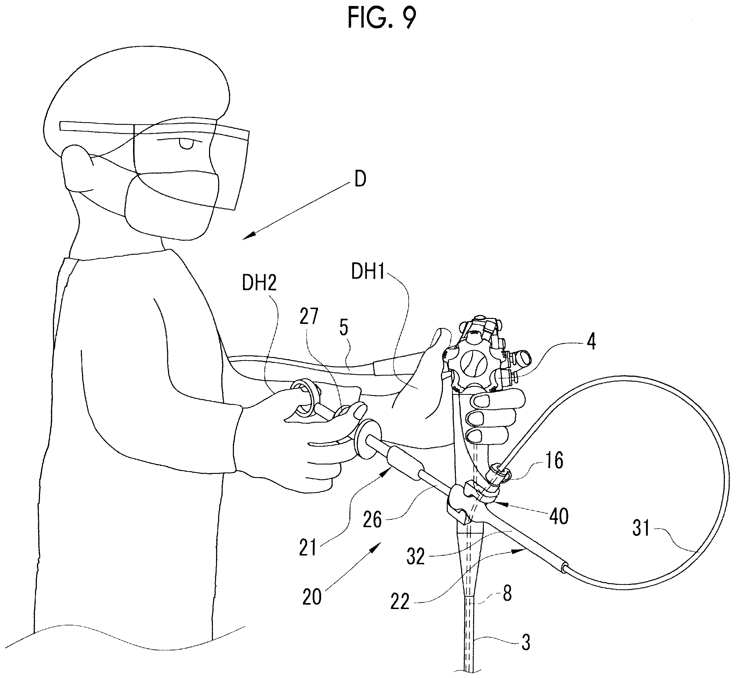

An operation using the endoscope system 1 in a case where the doctor D, who is the user, performs a treatment by the treatment tool device 20 for an endoscope will be described. First, the doctor D inserts the insertion part 3 of the endoscope 2 into the body of the patient P, observes an endoscope image captured by the image sensor 10, finds a region of interest such as a lesion portion 50 (see Fig. 10), and determines a portion to be treated. As shown in Fig. 9, first, while holding a state where the flexible sheath 23 and the hard part 26 are inserted into the oversheath 22, the doctor D inserts the oversheath 22 and the flexible sheath 23 into the forceps channel 8. In this case, the doctor D brings the snare wire 25 into the contracted state by the operation of the operation part 27. Next, the doctor D attaches the oversheath 22 to the operation part 4 of the endoscope 2 by engaging the oversheath with the attaching/detaching member 40.

As described above, the treatment tool main body 21 is slidable and rotatable with respect to the oversheath 22 within a range in which the hard part 26 and the fitting portions 31B and 32B are fitted to each other. That is, in a case where the oversheath 22 and the treatment tool main body 21 are inserted into the forceps channel 8, the treatment tool main body 21 can be slid and rotated within a certain range with respect to the forceps channel 8. Thus, the doctor D can slide the treatment tool main body 21 with respect to the oversheath 22 and align the distal end portion of the flexible sheath 23 with the position of the lesion portion 50, while observing the endoscope image captured by the image sensor 10 (state shown in Fig. 10A). In a case of performing this operation, the doctor D can perform the operation by grasping the operation part 4 of the endoscope 2 with one hand DH1 and grasping the treatment tool main body 21 with the other hand DH2.

Next, the operation part 27 is operated by the other hand DH2 to cause the snare wire 25 to protrude, or the treatment tool main body 21 is rotated with respect to the oversheath 22 to align the snare wire 25 with the position of the lesion portion 50 (state shown in Fig. 10B). Further, the doctor D operates the endoscope 2 with one hand DH1, for example, to bend the bending portion 3b. Thus, the position of the snare wire 25 is brought close to the position of the lesion portion 50, and the lesion portion 50 is surrounded by a loop of the snare wire 25 (state shown in Fig. 11A). Then, the doctor D can resect the lesion portion 50 from the body of the patient P by operating the high-frequency power supply 19 to cause a high-frequency current to flow through the snare wire 25 (state shown in Fig. 11B).

As described above, in a case where the treatment tool device 20 for an endoscope and the endoscope system 1 including the treatment tool device 20 for an endoscope are used, the doctor D can perform, in addition to the operation of the endoscope 2, a plurality of types of treatments such as an operation of sliding and rotating the treatment tool main body 21 with respect to the forceps channel 8, and the contraction and expansion of the snare wire 25 by himself or herself. Thus, it is possible to operate the treatment tool without the need for cooperation, and in a case where the doctor is a main operator, the number of people who perform the treatment can be reduced.

Since the oversheath 22 can be attached to the operation part 4 via the attaching/detaching member 40, the operation performed by the doctor D is concentrated near the operation part 4, and the operation of the treatment tool main body 21 can be stably performed. Furthermore, since the first resistance force R1 in a case where the oversheath 22 is advanced and retreated to and from the forceps valve 16 is greater than the second resistance force R2 in a case where the treatment tool main body 21 is advanced and retreated to and from the oversheath 22, the movement of the oversheath 22 can be regulated in a case where the treatment tool main body 21 is operated. Thus, the operation of the treatment tool main body 21 can be performed more stably.

### Second Embodiment

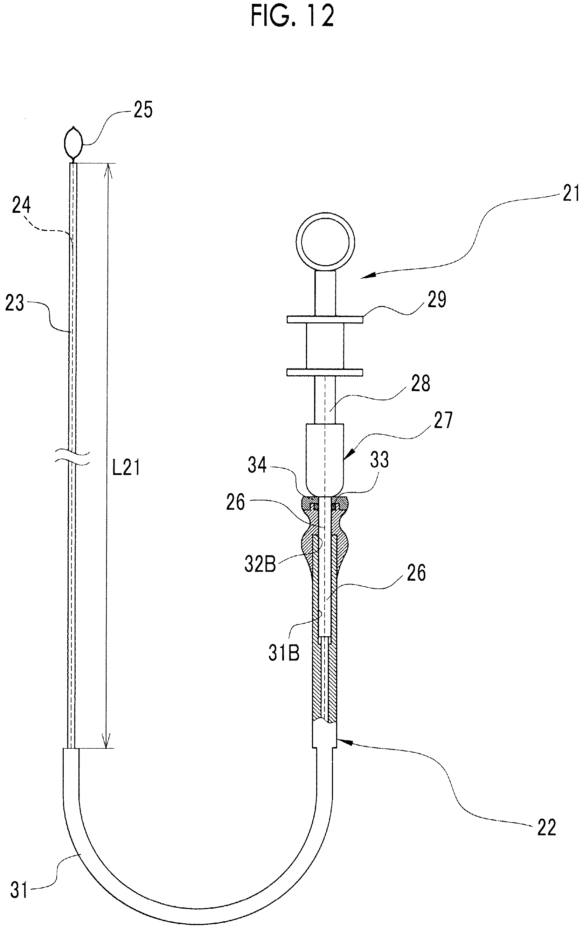

In a second embodiment to be described below, in addition to the configuration of the first embodiment, a length of the flexible sheath 23 protruding from the distal end of the oversheath 22 is specified. As shown in Fig. 12, in a case where a distal end of the operation part 27 of the treatment tool main body 21 is made to abut on the base end of the oversheath 22, that is, in a case where the treatment tool main body 21 is slid to a position where it abuts against the oversheath 22, a length L21 of the flexible sheath 23 protruding from the distal end of the oversheath 22 is 40 cm or more and 270 cm or less. The configurations other than the specification of the length L21 are the same as those of the first embodiment, and the description thereof will be omitted.

The length L21 described above is determined based on a length L22 from the forceps outlet 7 of the forceps channel 8 to a branch part 8A. That is, in a case where the distal end of the operation part 27 of the treatment tool main body 21 is made to abut on the base end of the oversheath 22, the length L21 of the flexible sheath 23 protruding from the distal end of the oversheath 22 is preferably longer than the length L22 from the forceps outlet 7 of the forceps channel 8 to the branch part 8A. The branch part 8A is a position where the forceps channel 8 and the suction channel 17 are joined together. Thus, it is possible to prevent the oversheath 22 inserted into the forceps channel 8 from reaching the position of the branch part 8A. Therefore, an outer diameter of the treatment tool main body 21 in the forceps channel 8 is smaller than a portion covered with the oversheath 22. Accordingly, the suction from the forceps channel 8 is not hindered.

In a case where the oversheath 22 inserted into the forceps channel 8 reaches the position of the branch part 8A, the suction from the forceps channel 8 is hindered because the oversheath 22 having a larger outer diameter than the flexible sheath 23 blocks the forceps channel 8. However, in the present embodiment, as described above, in a case where the distal end of the operation part 27 of the treatment tool main body 21 is made to abut on the base end of the oversheath 22, the length L21 of the flexible sheath 23 protruding from the distal end of the oversheath 22 is specified, so that the suction from the forceps channel 8 is not hindered.

### First Modification Example

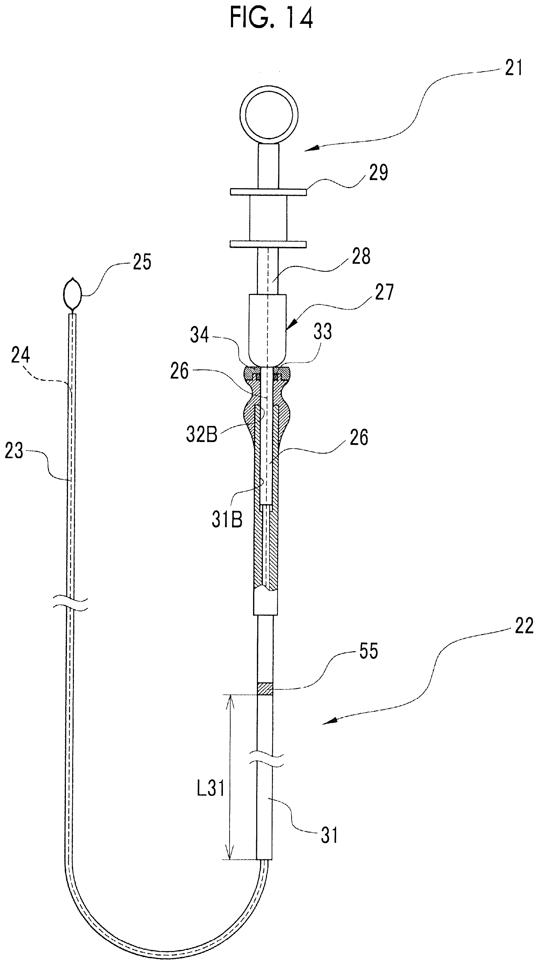

As a modification example of the second embodiment, as shown in Fig. 14, an index 55 indicating an insertion length of the oversheath 22 inserted into the forceps channel 8 may be provided on the oversheath 22. The index 55 is a mark provided at a position of a predetermined insertion length L31 from a distal end of the oversheath main body 31. That is, in a case where the oversheath 22 is inserted to the position of the index 55 with respect to the forceps channel 8, the mark indicates that the oversheath 22 is inserted to the predetermined insertion length L31.

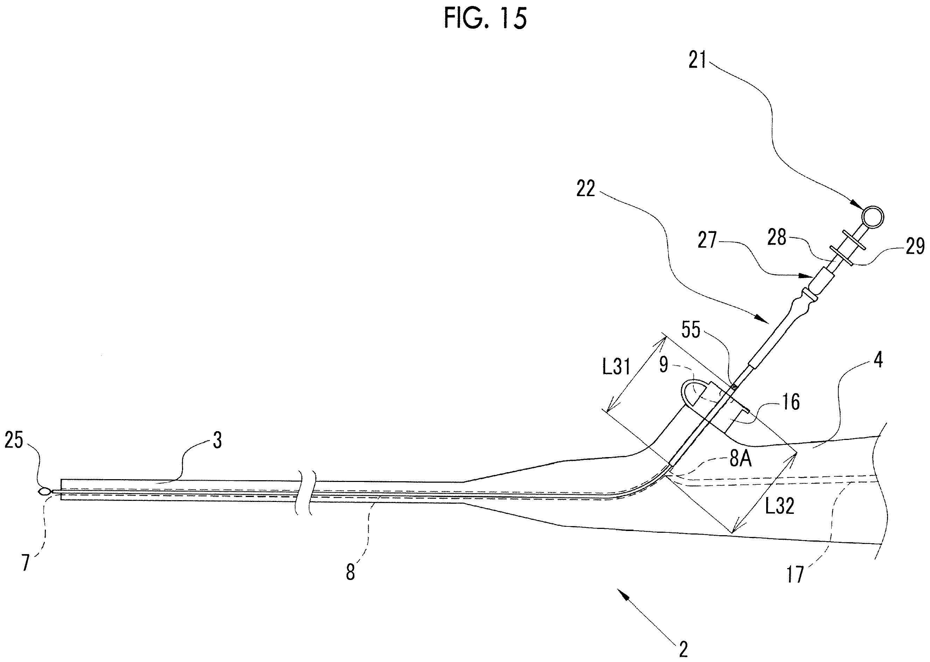

As shown in Fig. 15, the insertion length L31 indicating the position where the index 55 is provided is equal to or less than a length L32 from the forceps valve 16 of the forceps channel 8 to the branch part 8A. For example, the insertion length L31 is 4 cm. That is, in a case where the oversheath 22 is inserted to the position of the index 55 with respect to the forceps channel 8, the user can recognize that the oversheath 22 is inserted to or near the position of the branch part 8A. Thus, the user can adjust the insertion length of the oversheath 22 so that the treatment tool device 20 for an endoscope is connected to the endoscope 2, that is, inserted into the forceps channel 8 in a state where the oversheath 22 inserted into the forceps channel 8 does not reach the position of the branch part 8A. Therefore, the suction from the forceps channel 8 by the suction channel 17 is not hindered.

### Second Modification Example

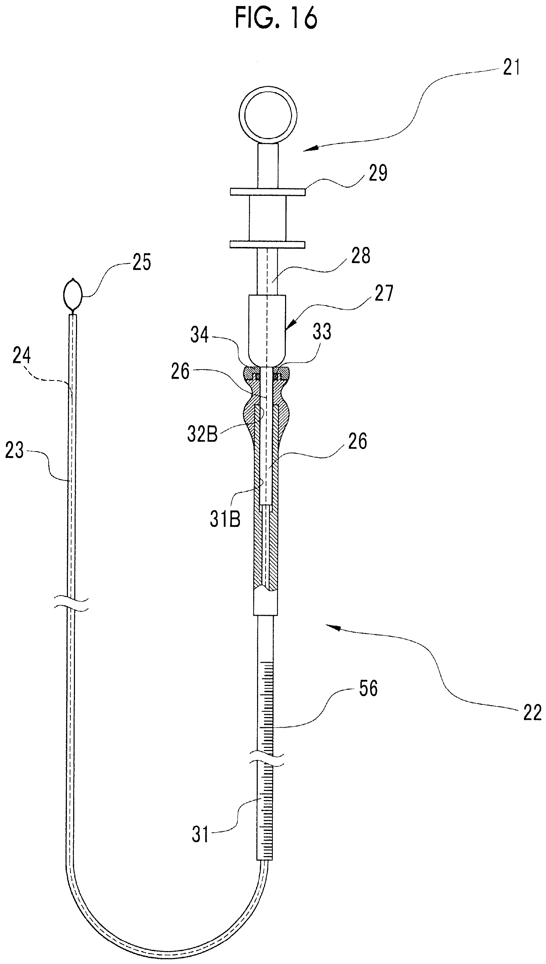

The index indicating the insertion length of the oversheath 22 inserted into the forceps channel 8 is not limited to the above modification example, and as shown in Fig. 16, gradations 56 indicating the insertion length of the oversheath 22, that is, a dimension from the distal end of the oversheath main body 31, may be provided as the index. Thus, the doctor or the assistant can set the treatment tool device 20 for an endoscope to be connected to the endoscope 2, that is, inserted into the forceps channel 8 in a state where the oversheath 22 does not reach the branch part 8A, while viewing the index 56 indicating the insertion length. Therefore, the same effect as that of the first modification example can be obtained.

### Third Modification Example

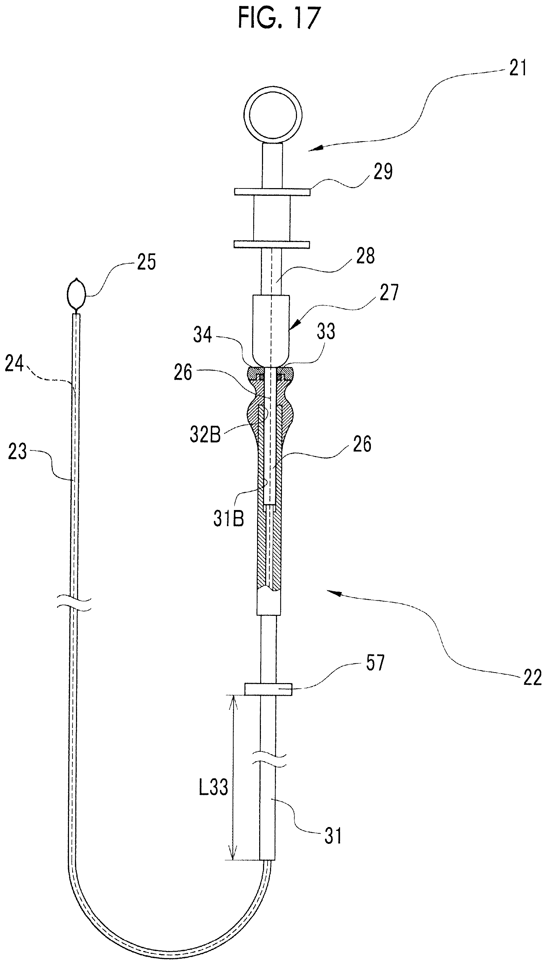

As shown in Fig. 17, a stopper 57 may be provided at a position of a predetermined dimension L33 from the distal end of the oversheath main body 31 instead of the index indicating the insertion length of the oversheath 22 inserted into the forceps channel 8. The predetermined dimension L33 is 4 cm. The stopper 57 is provided integrally with the oversheath main body 31, and protrudes from an outer peripheral surface of the oversheath main body 31. Thus, in a case where the oversheath main body 31 is inserted into the forceps channel 8, the stopper 57 abuts on the forceps valve 14 to regulate the insertion of the oversheath 22. That is, the oversheath 22 can be prevented from being inserted beyond a position where the stopper 57 abuts on the forceps valve 14. The doctor or the assistant can make the stopper 57 abut on the forceps valve 14 so that the treatment tool device 20 for an endoscope is connected to the endoscope 2, that is, inserted into the forceps channel 8 in a state where the oversheath 22 does not reach the branch part 8A. Therefore, the same effect as those of the first and second modification examples can be obtained.

### Fourth Modification Example

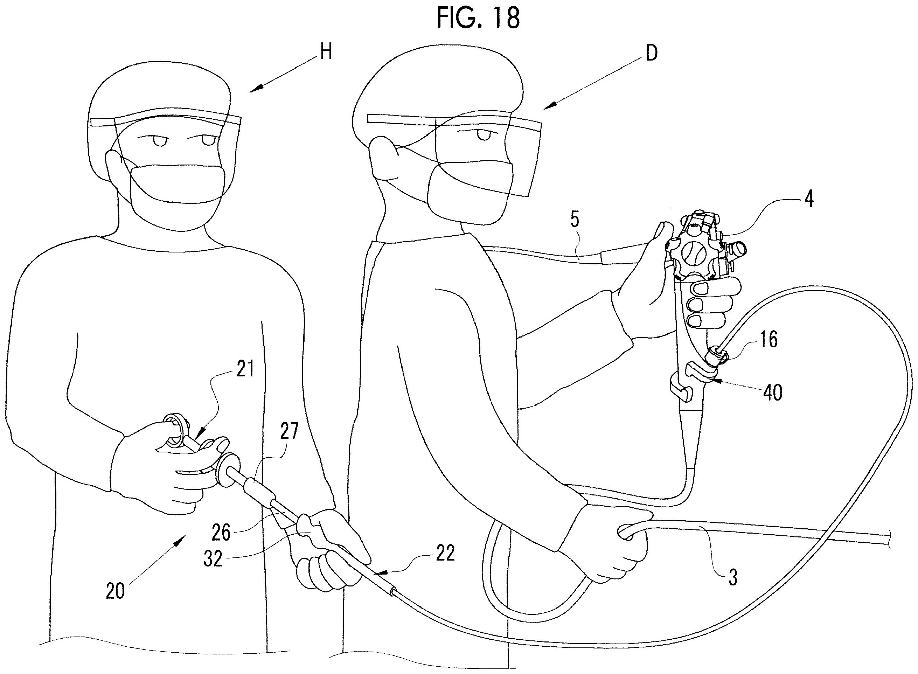

In the first and second embodiments, the example in which the doctor D performs a plurality of types of treatments by himself or herself is given, but the present invention is not limited to this, and the treatment may be performed by the doctor D and the assistant H. For example, as shown in Fig. 18, in a case where the doctor D operates the endoscope 2 with both hands, the assistant H can operate the treatment tool device 20 for an endoscope. That is, according to the configuration of the treatment tool device 20 for an endoscope in the first and second embodiments, the assistant H can perform a plurality of types of treatments such as an operation of sliding and rotating the treatment tool main body 21 with respect to the forceps channel 8, the contraction and expansion of the snare wire 25, and an operation of energizing the snare wire 25 by the high-frequency power supply 19. That is, the number of the types of treatment tool operations that can be performed by the assistant increases, and the treatment tool operations can be performed without the need for cooperation. As described above, it is possible to cope with not only a case where the doctor D performs the treatment by himself or herself but also the treatment by a plurality of people. In this case, it is preferable to detach the oversheath 22 from the operation part 4 of the endoscope 2 by releasing engagement with the attaching/detaching member 40 so that the assistant H can freely handle the oversheath 22 and the treatment tool main body 21.

### Fifth Modification Example

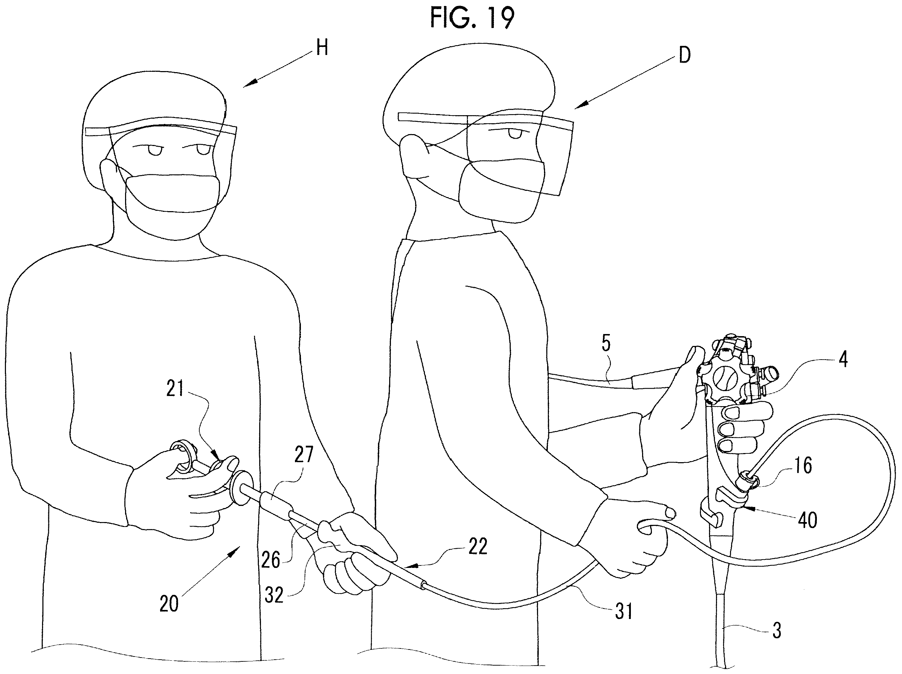

As shown in Fig. 19, the doctor D and the assistant H may cooperate with each other to perform the treatment. For example, the doctor D operates the endoscope 2, inserts the oversheath 22 into the forceps channel 8, and energizes the snare wire 25 by the high-frequency power supply 19, and the assistant H performs the operation of sliding and rotating the treatment tool main body 21 with respect to the forceps channel 8 and performs the contraction and expansion of the snare wire 25, thereby performing operations related to the treatment tool device 20 for an endoscope in a shared manner. In this case, it is preferable to detach the oversheath 22 from the operation part 4 of the endoscope 2 by releasing engagement with the attaching/detaching member 40 so that the assistant H can freely handle the oversheath 22 and the treatment tool main body 21.

In each of the above embodiments, as the treatment tool main body 21, a high-frequency snare comprising the snare wire 25 serving as a treatment part and expanding in a loop shape in a case of protruding from the distal end of the flexible sheath 23 by the push-pull operation of the operation wire 24 is exemplified. However, the treatment tool main body 21 is not limited thereto, and need only comprise at least the flexible sheath 23, the operation wire 24 inserted through the flexible sheath 23, the hard part 26 provided at the base end portion of the flexible sheath 23, and the operation part 27 continuously provided at the hard part 26, and may be, for example, a puncture needle or a treatment tool comprising an ultrasound oscillator. Further, it is preferable to comprise a treatment part that expands by the push-pull operation of the operation wire 24, and for example, forceps for holding biological tissue in the body, a clutch cutter for energizing the forceps with a high-frequency current, or the like may be applied as the treatment tool main body 21. In addition, in each of the above embodiments, as the endoscope, an upper digestive tract endoscope is exemplified. However, the endoscope is not limited thereto, and need only be an endoscope comprising a forceps valve, and may be, for example, a bronchoscope or a lower digestive tract endoscope.

### Appendix 1

A method of mounting a treatment tool device for an endoscope, the method comprising: setting, by a doctor or an assistant, the treatment tool device for an endoscope to be connected to the endoscope in a state where an oversheath does not reach a branch part between a forceps channel and a suction channel while viewing an index indicating an insertion length.

### Explanation of References

- 1:: endoscope system
- 2:: endoscope
- 3:: insertion part
- 3a:: distal end rigid portion
- 3b:: bending portion
- 3c:: flexible tube portion
- 4:: operation part
- 4A:: operation button
- 5:: universal cord
- 5A:: connector
- 7:: forceps outlet
- 8:: forceps channel
- 8A:: branch part
- 9:: forceps port
- 10:: image sensor
- 11:: processor device
- 12:: light source device
- 13:: display
- 14:: user interface (UI)
- 15:: mouthpiece for endoscope
- 16:: forceps valve
- 17:: suction channel
- 18:: suction device
- 19:: high-frequency power supply
- 20:: treatment tool device for endoscope
- 21:: treatment tool main body
- 22:: oversheath
- 23:: flexible sheath
- 24:: operation wire
- 25:: snare wire
- 26:: hard part
- 27:: operation part
- 28:: operation part main body
- 28A:: finger hook portion
- 28B:: cylindrical portion
- 29:: slider
- 31:: oversheath main body
- 31A:: pipe line
- 31B:: fitting portion
- 32:: seal holding member
- 32A:: recess portion
- 32B:: fitting portion
- 32C:: opening portion
- 32D:: engaging projection portion
- 33:: seal member
- 34:: lid member
- 34A:: through-hole
- 34B:: engaging recess portion
- 40:: attaching/detaching member
- 40A:: projection portion
- 50:: lesion portion
- 55:: index
- 56:: gradations
- 57:: stopper
- D:: doctor
- DH1:: one hand
- DH2:: the other hand
- H:: assistant
- L11, L12, L13, L21, L22, L31, L32, L33:: length
- M:: mouth
- P:: patient
- R1:: first resistance force
- R2:: second resistance force
- Z:: insertion direction

## Claims

1. A treatment tool device for an endoscope comprising:
a treatment tool main body that includes a flexible sheath having a tubular shape and inserted into a forceps channel of an endoscope, an operation wire inserted through the flexible sheath, a hard part provided at a base end portion of the flexible sheath and harder than the flexible sheath, and an operation part continuously provided at the hard part; and
an oversheath that is inserted into the forceps channel in a state where the flexible sheath and the hard part are inserted into the oversheath.

2. The treatment tool device for an endoscope according to claim 1,
wherein the oversheath has a fitting part that is fitted to the hard part, and
the treatment tool main body is slidable and rotatable with respect to the oversheath within a range in which the hard part and the fitting part are fitted to each other.

3. The treatment tool device for an endoscope according to claim 1 or 2,
wherein a length of the oversheath is shorter than a length of the flexible sheath.

4. The treatment tool device for an endoscope according to any one of claims 1 to 3,
wherein the oversheath is provided with a seal member filling a gap between the oversheath and the treatment tool main body at a base end portion.

5. The treatment tool device for an endoscope according to claim 4,
wherein the seal member is an annular elastic member housed inside the oversheath.

6. The treatment tool device for an endoscope according to any one of claims 1 to 5,
wherein, in a case where a distal end of the operation part is made to abut on a base end of the oversheath, a length of the flexible sheath protruding from a distal end of the oversheath is 40 cm or more and 270 cm or less.

7. The treatment tool device for an endoscope according to any one of claims 1 to 6,
wherein the oversheath is provided with an index indicating an insertion length of the oversheath inserted into the forceps channel.

8. The treatment tool device for an endoscope according to claim 7,
wherein a mark is used as the index indicating the insertion length, and a dimension from a distal end of the oversheath to the mark is 4 cm.

9. The treatment tool device for an endoscope according to claim 7,
wherein gradations indicating a dimension from a distal end of the oversheath are used as the index indicating the insertion length.

10. The treatment tool device for an endoscope according to any one of claims 1 to 6,
wherein the oversheath has an oversheath main body that is inserted into the forceps channel and a stopper provided on the oversheath main body to regulate insertion of the oversheath.

11. The treatment tool device for an endoscope according to claim 10,
wherein a dimension from a distal end of the oversheath main body to the stopper is 4 cm.

12. An endoscope system comprising:
an endoscope provided with a forceps channel and a forceps valve; and
the treatment tool device for an endoscope according to any one of claims 1 to 11.

13. An endoscope system comprising:
an endoscope provided with a forceps channel, a forceps valve, and a suction channel connected to the forceps channel; and
the treatment tool device for an endoscope according to any one of claims 7 to 11,
wherein the treatment tool device for an endoscope is inserted into the forceps channel in a state where the oversheath does not reach a branch part between the forceps channel and the suction channel.

14. The endoscope system according to claim 12 or 13,
wherein the endoscope is provided with an attaching/detaching member that makes the oversheath attachable and detachable.

15. The endoscope system according to any one of claims 12 to 14,
wherein a first resistance force in a case where the oversheath is advanced and retreated to and from the forceps valve is greater than a second resistance force in a case where the treatment tool main body is advanced and retreated to and from the oversheath.
